# EUROPEAN PATENT SPECIFICATION

(11) **EP 3 229 023 B1**
(45) Date of publication and mention of the grant of the patent: **07.06.2023**
(21) Application number: 15865332.9
(22) Date of filing: 06.10.2015
(51) Int. Cl.: G01N 33/53, G01N 33/68, G01N 33/543

(54) **METHOD FOR DETECTING AGGREGATE FORM OF AGGREGATE-FORMING POLYPEPTIDE**
VERFAHREN ZUR DETEKTION EINER AGGREGATFORM EINES AGGREGATBILDENDEN POLYPEPTIDS
PROCÉDÉ DE DÉTECTION D'UNE FORME AGRÉGÉE D'UN POLYPEPTIDE FORMANT DES AGRÉGATS

(30) Priority: 02.12.2014 KR 20140170608
(43) Date of publication of application: 11.10.2017
(73) Proprietor: Peoplebio Inc., Seongnam-si, 13487 (KR)
(72) Inventor: LEE, Byoung Sub, Anyang-si Gyeonggi-do 14027 (KR); LEE, Kwan Soo, Seoul 06566 (KR); KIM, Shin Won, Seoul 05318 (KR); LIM, Kun Taek, Gyeonggi-do, 10872 (KR); KIM, Gwang Je, Incheon 22704 (KR); YU, Ji Sun, Seoul 06057 (KR)
(74) Representative: González Peces, Gustavo Adolfo
(86) International application number: PCT/KR2015/010558
(87) International publication number: WO 2016/088999

(56) References cited:
- EP-A1- 2 579 042
- WO-A1-2007/123345
- KR-A- 20100 036 324
- KR-A- 20110 081 330
- KR-A- 20140 043 371
- KR-A- 20140 069 346
- US-A1- 2010 009 388
- US-A1- 2010 062 540
- KANG S ET AL: "Blood Detection of Alzheimer'S Diseases With Multimer Detection System-ad", ALZHEIMER'S & DEMENTIA: THE JOURNAL OF THE ALZHEIMER'SASSOCIATION, ELSEVIER, NEW YORK, NY, US, vol. 6, no. 4, 1 July 2010 (2010-07-01), page e43, XP027440574, ISSN: 1552-5260, DOI: 10.1016/J.JALZ.2010.08.132 [retrieved on 2010-07-01]
- SangYun Kim, ET AL: "THE FIRST BLOOD-BASED BIOMARKER OF ALZHEIMER'S DISEASE WITH HIGH PREDICTABILITY: OLIGOMERIC BETA- AMYLOID DETECTION BY MULTIMER DETECTION SYSTEM", , 18 July 2013 (2013-07-18), XP055396974, Retrieved from the Internet: URL:http://www.alzheimersanddementia.com/a rticle/S1552-5260(13)02515-6/pdf [retrieved on 2017-08-08]
- DATABASE GENBANK [Online] 14 July 2000 XP055391999 Retrieved from NCBI Database accession no. AAB26264
- DATABASE PROTEIN [Online] 11 May 2014 XP055392000 Retrieved from NCBI Database accession no. NP_000336

## Description

### Technical Field

The present patent application claims priority to and the benefit of Korean Patent Application No. 10-2014-0170608, filed in the Korean Intellectual Property Office on 02 December 2014.

The present invention relates to a method for detecting an aggregate form of an aggregate-forming polypeptide in a biosample as defined in the appended set of claims.

### Background Art

First, in some cases, polypeptides constituting proteins make functional proteins by forming multimers. However, when polypeptides present as monomers in a normal state form multimers, they aggregate abnormally (e.g., being converted into a misfolded form), and cause diseases (Massimo Stefani, et al., J. Mol. Med. 81:678-699(2003); and Radford SE, et al., Cell. 97:291-298(1999)) .

For example, the diseases or disorders associated with abnormal aggregation or misfolding of proteins include Alzheimer's disease, Creutzfeldt-Jakob disease, spongiform encephalopathies, Parkinson's disease, Huntington's disease, amyotrophic lateral sclerosis, Serpin deficiency, emphysema, cirrhosis, type II diabetes, primary systemic amyloidosis, secondary systemic amyloidosis, frontotemporal dementias, senile systemic amyloidosis, familial amyloid polyneuropathy, hereditary cerebral amyloid angiopathy, and haemodialysis-related amyloidosis.

In measuring the presence or absence or the progress of such diseases or disorders, when such measurement is difficult since the amount of the antigen is very small in the sample or the size of the antigen is very small, or when the amount of the antigen in the body is not proportional to the amount of the antigen in the sample, for example, (although the level of Aβ (amyloid-beta), which is implicated in Alzheimer's disease, is known to be higher in an abnormal person than in a normal person), when the amount of the Aβ oligomer in a blood sample is difficult to detect or the Aβ oligomer exits atypically in the blood sample, diagnosis may be difficult.

In addition, the antigen to be measured is too small in size or too small in amount, and thus, the diagnosis of diseases is often not easy by sandwich ELISA.

Kang et al ("Blood detection of Alzheimer's diseases with multimer detection system-ad") ALZHEIMER'S & DEMENTIA: THE JOURNAL OF THE ALZHEIMER'S ASSOCIATION, ELSEVIER, NEW YORK, NY, US, vol. 6, no. 4, 1 July 2010 (2010-07-01), page e43) discloses a multimer detection system for Alzheimer's disease that is tested against spiked synthetic amyloid beta oligomers in blood. Plasma samples from patients having Alzheimer's disease and from healthy control are analysed.

WO 2007/123345 A1 discloses a method for differentially detecting a multimeric form from a monomeric form of a multimer-forming polypeptide in a biosample. For this, a plasma sample that comprises a multimer forming polypeptides is prepared and a capturing antibody bound to a magnetic bead is added to the sample. A detection antibody is also added to the sample and the multimer is detected by ECL. Detection of amyloid-13 and α-synuclein is disclosed.

EP 2579042 A1 discloses a method for detecting Aβ-specific antibodies in a biological sample, especially in connection with and for diagnosing of Alzheimer's disease. In particular, the method comprises contacting the sample with Aβ-aggregates and allowing the Aβ-specific antibodies to bind to the Aβ-aggregates, and detecting the Aβ-specific antibodies bound to the Aβ-aggregates by a single particle detection technique.

US 2010/009388 A1 discloses a method for differentially detecting a polypeptide in a biosample. The method comprises the steps of mixing plasma sample with the human recombinant PrP in the presence of plasminogen inhibitors and incubated for 15 minutes at room temperature and afterwards the samples are applied to MDS and incubated again for 1h before performing the measurement.

Accordingly, the present inventors recognized the need for the development of a method for detecting an aggregate form of an aggregate-forming polypeptide, the method maximizing a differentiation in the diagnostic signal between a patient and a normal subject.

### Summary of the invention

The present invention is set forth in the appended set of claims.

### Detailed Description of the Invention

### Technical Problem

Under the above background, the present inventors have conducted extensive research to develop a novel method for detecting an aggregate form of an aggregate-forming polypeptide, and as a result have developed a method for detecting an aggregate form of an aggregate-forming polypeptide, the method maximizing a difference in the diagnostic signal between a patient and a normal subject using a difference in the clearing system suppressing the formation of an aggregate form of a polypeptide or a difference in hydrophobic interaction.

Therefore, an aspect of the present invention is to provide a method for detecting an aggregate form of an aggregate-forming polypeptide in a biosample as defined in the appended set of claims.

Other purposes and advantages of the present invention will become more obvious with the following detailed description of the invention, claims, and drawings.

### Technical Solution

In accordance with an aspect of the present invention, there is provided a method for detecting an aggregate form of an aggregate-forming polypeptide in a biosample, the method including the steps of: (a) spiking, with a biosample to be analyzed, (i) a monomeric or multimeric form of the aggregate-forming polypeptide, (ii) a hydrophobic deleted derivative of the aggregate-forming polypeptide, or (iii) a monomeric or multimeric form of the aggregate-forming polypeptide and a hydrophobic deleted derivative of the aggregate-forming polypeptide; (b) additionally forming an aggregate form of the aggregate-forming polypeptide by incubating a product of step (a) at 25-40°C for 2-6 days; (c) contacting, with a product of step (b), a binder-label in which a signal generation label is conjugated to a binder binding to the aggregate form of the aggregate-forming polypeptide; and (d) detecting a signal generated from the binder-label bound to the aggregate form of the aggregate-forming polypeptide, wherein the incubating in step (b) is carried out for a sufficient incubation time for multimerization of the spiked (i), (ii), or (iii) by the biosample, wherein the biosample is plasma, and wherein the biosample for performing the multimerization of the spiked (i), (ii), or (iii) is a biosample of a human being having a disease involving the multimeric form of the aggregate-forming polypeptide.

The present invention is directed to a method for detecting an aggregate form of an aggregate-forming polypeptide, the method maximizing a difference in the diagnostic signal between a patient and a normal subject using a difference in the clearing system suppressing the formation of an aggregate form of a polypeptide or a difference in hydrophobic interaction.

As used herein, the term "aggregate-forming polypeptide" refers to a polypeptide capable of forming a multimeric form (oligomeric form) or forming an aggregate form through hydrophobic interaction with a monomer. In particular, the structural changes above cause various diseases. For example, the diseases include Alzheimer's disease, Creutzfeldt-Jakob disease, spongiform encephalopathies, Parkinson's disease, Huntington's disease, amyotrophic lateral sclerosis, Serpin deficiency, emphysema, cirrhosis, type II diabetes, primary systemic amyloidosis, secondary systemic amyloidosis, frontotemporal dementias, senile systemic amyloidosis, familial amyloid polyneuropathy, hereditary cerebral amyloid angiopathy, and haemodialysis-related amyloidosis.

Generally, non-monomeric forms of the aggregate-forming polypeptide are normal, but an aggregate form thereof causes a neurodegenerative disease, such as, especially Alzheimer's disease, Creutzfeldt-Jakob disease, or Parkinson's disease.

Hereinafter, the method of the present invention for detecting an aggregate form of an aggregate-forming polypeptide in a biosample will be described in detail step by step.

### (a) Step of spiking

First, the method of the present invention includes spiking, with a biosample to be analyzed, (i) a monomeric or multimeric form (oligomeric form) of the aggregate-forming polypeptide, (ii) a hydrophobic deleted derivative of the aggregate-forming polypeptide, or (iii) a monomeric or multimeric form (oligomeric form) of the aggregate-forming polypeptide and a hydrophobic deleted derivative of the aggregate-forming polypeptide.

As used herein, the term "biosample" refers to an organism-originated sample to be analyzed. The biosample is plasma.

According to another embodiment of the present invention, the aggregate-forming polypeptide include Aβ peptide and tau protein involved in Alzheimer's disease, prion involved in Creutzfeldt-Jakob disease and sponge foam brain disease, α-synuclein involved in Parkinson's disease, in Ig light chain involved in primary systemic amyloidosis, serum amyloid A involved in secondary systemic amyloidosis, tau protein involved in frontotemporal dementias, transthyretin involved in senile systemic amyloidosis, transthyretin involved in familial amyloid multiple neuropathy, cystatin C involved in hereditary cerebral amyloid angiopathy, β2-microglobulin involved in haemodialysis-related amyloidosis, Huntingtin involved in Huntington's disease, superoxide dismutase involved in amyotrophic lateral sclerosis, serpin involved in serpin deficiency, pulmonary emphysema, and cirrhosis, and amylin involved in type II diabetes. More preferably, the aggregate-forming polypeptide is Aβ peptide or tau protein involved in Alzheimer's disease, or α-synuclein involved in Parkinson's disease, most preferably, Aβ peptide or α-synuclein.

As used herein, the term "spiking" refers to a procedure of adding to or adding to and then mixing with a biosample to be analyzed, a monomeric form (or multimeric form) of an aggregate-forming polypeptide and/or a hydrophobic deleted derivative of an aggregate-forming polypeptide.

As used herein, the term "multimer" is one that is formed through a combination of two or more monomers, and also includes an oligomer.

According to the present invention, in cases where (i) a monomeric or multimeric form of the aggregate-forming polypeptide is spiked with a biosample to be analyzed, the difference in the diagnostic signal between a patient and a normal subject is intended to be maximized using a difference in the clearing system suppressing the formation of an aggregate form of an aggregate-forming polypeptide, that is, a biosample of the patient has a low degree of the clearing system, promoting the formation of an aggregate form of an aggregate-forming polypeptide, but a biosample of a normal subject has a high degree of the clearing system, reducing the formation of an aggregate form of a aggregate-forming polypeptide, thereby maximizing the difference in the diagnostic signal.

According to still another embodiment of the present invention, the monomeric form of the aggregate-forming polypeptide is Aβ peptide including the amino acid sequence of SEQ ID NO: 1 or α-synuclein including the amino acid sequence of SEQ ID NO: 2.

According to the present invention, in cases where (ii) a hydrophobic deleted derivative of the aggregate-forming polypeptide is spiked with a biosample to be analyzed, the difference in the diagnostic signal between a patient and a normal subject is intended to be maximized using a difference in hydrophobic interaction between a hydrophobic deleted derivative of an aggregate-forming polypeptide including hydrophobic amino acid residues and a monomeric or multimeric form (oligomeric form) of an aggregate-forming polypeptide existing in the biosample, that is, a monomeric or multimeric form (oligomeric form) of an aggregate-forming polypeptide existing in the biosample of the patient and a hydrophobic deleted derivative of an aggregate-forming polypeptide form a large amount of aggregate forms through hydrophobic interaction, or a monomeric form of an aggregate-forming polypeptide existing in a biosample of a normal subject and a hydrophobic deleted derivative of an aggregate-forming polypeptide form aggregate forms through hydrophobic interaction, but form a smaller amount of aggregate forms compared with a patient, thereby maximizing the difference in the diagnostic signal between the patient and the normal subject.

As used herein, the term "hydrophobic deleted derivative of an aggregate-forming polypeptide" refers to a derivative, in which amino acid residues are deleted to include a plurality of hydrophobic amino acid residues in the amino acid sequence of an aggregate-forming polypeptide so as to form an aggregate form through a hydrophobic interaction with a monomeric or multimeric form (oligomeric form) of an aggregate-forming polypeptide.

The hydrophobic deleted derivative of the aggregate-forming polypeptide may be selected in consideration of the length (molecular weight) and/or hydrophobic amino acid residue for hydrophobic interaction with a monomeric or multimeric form (oligomeric form) of an aggregate-forming polypeptide. Preferably, the hydrophobic deleted derivative of the aggregate-forming polypeptide is Aβ_{delete} peptide including the 37th to 42nd amino acid residues in the amino acid sequence of SEQ ID NO: 1. More preferably, the hydrophobic deleted derivative of the aggregate-forming polypeptide is Aβ_{delete} peptide including the 29th to 42nd amino acid residues in the amino acid sequence of SEQ ID NO: 1. Still more preferably, the hydrophobic deleted derivative of the aggregate-forming polypeptide is Aβ_{delete} peptide including the 17th to 42nd amino acid residues in the amino acid sequence of SEQ ID NO: 1. Most preferably, the hydrophobic deleted derivative of the aggregate-forming polypeptide is Aβ_{delete} peptide including the 9th to 42nd amino acid residues in the amino acid sequence of SEQ ID NO: 1.

According to the present invention, in cases where (iii) a monomeric or multimeric form of the aggregate-forming polypeptide and a hydrophobic deleted derivative of the aggregate-forming polypeptide are spiked with a biosample to be analyzed, both effects attained by spiking (i) the monomeric or multimeric form of the aggregate-forming polypeptide and (ii) the hydrophobic deleted derivative of the aggregate-forming polypeptide, respectively, are intended to be employed, that is, the difference in the diagnostic signal between a patient and a normal subject is intended to be maximized using the difference in the clearing system suppressing the formation of an aggregate form of a polypeptide and the difference in hydrophobic interaction.

According to another embodiment of the present invention, a buffer is additionally added to the product in step (a). More preferably, the buffer is added in an amount of 3-15 times (v/v) to a biosample, still more preferably, 5-13 times (v/v), yet more preferably, 7-11 times (v/v), and even more preferably 8-10 times (v/v).

For the buffer used in the present invention, various buffers known in the art may be used, but preferably, the buffer is a non-ionic surfactantcontaining phosphate buffer.

For the non-ionic surfactant contained in the phosphate buffer used in the present invention, various non-ionic surfactants known in the art may be used, and preferably the non-ionic surfactant includes alkoxylated alkyl ethers, alkoxylated alkyl esters, alkyl polyglycosides, polyglyceryl esters, polysorbates, and sugar esters. More preferably, Tween-20 or Triton X-100 is used, and most preferably, Tween-20 is used.

### (b) Step of additionally forming aggregate form of aggregate-forming polypeptide

Then, the method of the present invention includes step (b) of additionally forming an aggregate form of the aggregate-forming polypeptide by incubating the product of step (a).

Here, one of the greatest features of the present invention is that, in cases where the measurement is difficult since the amount of an aggregate form of an aggregate-forming polypeptide (antigen) to be measured is very small in a biosample or the size of an aggregate form of an aggregate-forming polypeptide is very small, or in cases where the amount of the aggregate form of the aggregate-forming polypeptide (antigen) in the body is not proportional to the amount of the aggregate form of the aggregate-forming polypeptide (antigen) in the biosample, (i) the monomeric or multimeric form of the aggregate-forming polypeptide, (ii) the hydrophobic deleted derivative of the aggregate-forming polypeptide, or (iii) the monomeric or multimeric form of the aggregate-forming polypeptide and the hydrophobic deleted derivative of the aggregate-forming polypeptide are spiked with the biosample to additionally form an aggregate form of the aggregate-forming polypeptide, so that the presence or absence or the progress of a disease or disorder can be measured.

According to another embodiment of the present invention, the additional forming of the aggregate form of the aggregate-forming polypeptide in step (b) is conducted by incubating the production in step (a) at a temperature of 25-40°C, and even more preferably 25-38°C.

According to another embodiment of the present invention, in order to incubate for a time sufficient for a signal generated using a human biosample to be 1.5-20 times greater than a signal generated using a biosample of a normal subject, the additional formation of the aggregate form of the aggregate-forming polypeptide in step (b) is conducted by incubation of the production in step (a) for 2 days to 6 days, even more preferably for 3 days to 6 days, yet even more preferably for 4 days to 6 days, and most preferably for 5 days to 6 days.

As used herein, the term "incubation" refers to standing or shaking a biosample to be analyzed at a predetermined temperature for a predetermined period of time, and such shaking is, preferably, mild shaking.

Another of the greatest features of the present invention is that a biosample is allowed to stand (i.e., incubation) at a predetermined temperature for a predetermined period of time, so that the monomeric form (or multimeric form) of the aggregate-forming polypeptide and/or the hydrophobic deleted derivative of the aggregate-forming polypeptide and the aggregate-forming polypeptide, which exist in the biosample, aggregate well together, thereby maximizing the difference in the diagnostic signal between a patient and a normal subject.

### (c) Contacting, with product in step (b), a binder (binding to aggregate form of aggregate-forming polypeptide)-label

Then, the method of the present invention includes step (c) of contacting, with a production of step (b), a binder-label in which a signal generation label is conjugated to the binder binding to the aggregate form of the aggregate-forming polypeptide.

The binder binding to the aggregate form of the aggregate-forming polypeptide in the present invention includes an antibody, a peptide aptamer, an AdNectin, an affibody (USP No. 5,831,012), an avimer (Silverman, J. et al, Nature Biotechnology 23(12) :1556(2005)) or a Kunitz domain (Arnoux B et al., Acta Crystallogr. D Biol. Crystallogr. 58(Pt 7):12524(2002), and Nixon, AE, Current opinion in drug discovery & development 9(2):2618(2006)).

In the present invention, a signal generation label, which is conjugated to a binder binding to the aggregate form of the aggregate-forming polypeptide, includes a compound label (e.g., biotin), an enzyme label (e.g., alkaline phosphatase, peroxidase, β-galactosidase, and β-glucosidase), a radioactive label (e.g., I¹²⁵ and C¹⁴), a fluorescent label (e.g., fluorescein), a luminescent label, a chemiluminescent label, and a fluorescence resonance energy transfer (FRET) label, but is not limited thereto.

### (d) Detecting signal generated from binder-label binding to aggregate form of aggregate-forming polypeptide

Last, the method of the present invention includes step (d) of detecting a signal generated from the binder-label binding to the aggregate form of the aggregate-forming polypeptide.

The detecting of the signal generated from the binder-label binding to the aggregate form of the aggregate-forming polypeptide may be conducted by various methods known in the art, and for example, an immunoassay method associated with an antigen-antibody reaction may be used.

According to still another emobdiment of the present invention, steps (c) and (d) are performed by including the following steps: (c-1) contacting the product of step (b) with a capture antibody recognizing an epitope on the aggregate-forming polypeptide capturing the aggregate form; (c-2) contacting the captured aggregate form with a detection antibody recognizing an epitope on the aggregate-forming polypeptide; and (c-3) detecting an aggregate form-detection antibody complex.

Such a detection method employs two types of antibodies, namely, a capture antibody and a detection antibody. As used herein, the term "capture antibody" refers to an antibody that can bind to an aggregate-forming polypeptide to be detected in a biosample. The term "detection antibody" refers to an antibody that can bind to an aggregate-forming polypeptide captured by the capture antibody. The term "antibody" refers to an immunoglobulin protein that can bind to an antigen. The antibody used herein includes antibody fragments (e.g., F(ab')2, Fab', Fab, Fv) as well as a whole antibody that can bind to an epitope, an antigen, or an antigen fragment.

The detection method employs one set of a capture antibody and a detection antibody, which specifically recognizes epitopes on an aggregate-forming polypeptide, and the epitopes specifically recognized by the capture antibody and the detection antibody are identical to or overlapped with each other.

As used herein to recite the epitope with respect to the capture antibody and the detection antibody, the term "overlapped with" encompasses epitopes having completely or partially overlapped amino acid sequences. For example, the epitopes to 6E10 and WO2 antibodies have amino acid sequences including amino acids residue 3-8 and 4-10, respectively, of the human Aβ peptide sequence; the epitopes to 6E10 and FF51 antibodies have amino acid sequences including amino acids residues 3-8 and 1-4, respectively, of the human Aβ peptide sequence; the epitopes to 1E11 and WO2 antibodies have amino acid sequences including amino acid residues 1-8 and 4-10, respectively, of the human Aβ peptide sequence; and the epitopes to 1E11 and FF51 antibodies have amino acid sequences including amino acid residues 1-8 and 1-4, respectively, of the human Aβ peptide sequence. Such epitopes may be described as completely overlapped epitopes.

In addition, the epitopes to 3B6 and 3B6 biotin antibodies have sequences including amino acid residues 119-140 of the α-synuclein protein sequence.

As expressed herein to recite the human Aβ peptide sequence, the epitope has an amino acid sequence including amino acid residues 1-8, 3-8, 1-4, or 4-10; and, as expressed herein to recite the α-synuclein protein sequence, the epitope has an amino acid sequence including amino acids 119-140.

The epitope recognized by the capture antibody has a sequence that is not repeated in the aggregate-forming polypeptide, and the epitope recognized by the detection antibody has a sequence that is not repeated in the aggregate-forming polypeptide. According to the detection method of the present invention, the aggregate-forming polypeptide bound to the capture antibody cannot further bind to the detection antibody, and the reason is that there is no additional epitope recognized by the detection antibody.

The capture antibody and the detection antibody can be identical to each other. That is, the epitopes, specifically bound to the capture antibody and the detection antibody, are preferably identical to each other.

According to still another embodiment of the present invention, the capture antibody is bound to a solid substrate. Such a known material includes polystyrene, polypropylene, glass, metal, and a hydrocarbon polymer, such as a gel. The solid substrate may be present in the form of a dipstick, a microtiter plate, a particle (e.g., bead), an affinity column, and an immunoblot membrane (e.g., a polyvinylidene fluoride membrane) (see, USP 5,143,825, 5,374,530, 4,908,305, and 5,498,551).

According to another embodiment of the present invention, the detection antibody has a label generating a detectable signal. The label includes a compound label (e.g., biotin), an enzyme label (e.g., alkaline phosphatase, peroxidase, β-galactosidase, and β-glucosidase), a radioactive label (e.g., I¹²⁵ and C¹⁴), a fluorescent label (e.g., fluorescein), a luminescent label, a chemiluminescent label, and a fluorescence resonance energy transfer (FRET) label, but is not limited thereto. Various labels and methods for labeling antibodies are known in the art (Harlow and Lane, eds. Antibodies: A Laboratory Manual (1988) Cold Spring Harbor Laboratory Press, Cold Spring Harbor, N.Y.).

The antibodies that can be bound to aggregate-forming polypeptides may be prepared using epitopes that are conventionally described as immunogens according to the prior art, such as a fusion method (Kohler and Milstein, European Journal of Immunology, 6:511-519(1976)), a recombinant DNA method (USP 4,816,567), or a phage antibody library method (Clackson et al, Nature, 352:624-628(1991) and Marks et al, J. Mol. Biol., 222:58, 1-597(1991)). General methods for the production of antibodies are described in Harlow, E. and Lane, D., Antibodies: A Laboratory Manual, Cold Spring Harbor Press, New York, 1988; Zola, H., Monoclonal Antibodies: A Manual of Techniques, CRC Press, Inc., Boca Raton, Florida, 1984; and Coligan, CURRENT PROTOCOLS IN IMMUNOLOGY, Wiley/Greene, NY, 1991.

The preparation of hybridoma cell lines for the production of monoclonal antibodies is conducted by the fusion of an immortal cell line and antibody-producing lymphocytes. The preparation of monoclonal antibodies may be conducted using techniques known in the art. The polyclonal antibodies may be prepared by injecting the foregoing antigen into a suitable animal, collecting anti-serum containing an antibody, and then isolating the antibody by a method for isolating an antibody through a known affinity technique.

The detection of the aggregate form-detection antibody complex may be conducted by various methods known in the art. The formation of the aggregate form-detection antibody complex shows the presence of the aggregate form in the biosample. The step above may be quantitatively or qualitatively conducted using various detectable label/substrate pairs disclosed in, for example, Enzyme Immunoassay, E. T. Maggio, ed., CRC Press, Boca Raton, Florida, 1980 and Harlow and Lane, eds. Antibodies: A Laboratory Manual (1988) Cold Spring Harbor Laboratory Press, Cold Spring Harbor, N.Y., by the conventional method.

In cases where the detection antibody is labeled with alkaline phosphatase, bromochloroindolylphosphate (BCIP), nitro blue tetrazolium (NBT), or ECF may be used as a substrate for a color development reaction; in cases where the detection antibody is labeled with horseradish peroxidase, chloronaphthol, aminoethyl carbazole, diaminobenzidine, D-luciferin, lucigenin (bis-N-methylacridinium nitrate), resorufin benzyl ether, luminol, Amplex Red reagent (10-acetyl-3,7-dihydroxyphenoxazine), TMB (3,3,5,5-tetramethylbenzidine), enhanced chemiluminescence (ECL), or ABTS (2,2-azine-di[3-ethylbenzthiazoline sulfonate]) may be used as a substrate.

Through such a method, the signal generated using a biosample from a human being having a disease involving a multimeric form of an aggregate-forming polypeptide can be increased by 1.5-20 times compared with the signal generated using a biosample of a normal human being, preferably by 1.5-10 times, and more preferably by 1.6-10 times.

In accordance with another disclosure, not part of of the present invention, there is provided a kit for detecting an aggregate form of an aggregate-forming polypeptide in a biosample, the kit including: (i) a monomeric or multimeric form of the aggregate-forming polypeptide, (ii) a hydrophobic deleted derivative of the aggregate-forming polypeptide, or (iii) a monomeric or multimeric form of the aggregate-forming polypeptide and a hydrophobic deleted derivative of the aggregate-forming polypeptide.

The kit uses the foregoing method for detecting an aggregate form of an aggregate-forming polypeptide in a biosample, and thus the description of overlapping contents therebetween will be omitted to avoid excessive complexity of the specification due to repetitive descriptions thereof.

According to another disclosure, not part of the present invention, the kit further includes: a capture antibody recognizing an epitope on the aggregate-forming polypeptide; and a detection antibody recognizing the epitope recognized by the capture antibody.

### Advantageous Effects

Features and advantages of the present invention are summarized as follows:
(a) The present invention provides a method for detecting an aggregate form of an aggregate-forming polypeptide in a biosample.
(b) In the method of the present invention, in cases where the measurement is difficult since the amount of the aggregate form of the aggregate-forming polypeptide (antigen) to be measured is very small in a biosample or the size of the aggregate form of the aggregate-forming polypeptide (antigen) is very small, or in cases where the amount of the aggregate form of the aggregate-forming polypeptide (antigen) in the body is not proportional to the amount of the aggregate form of the aggregate-forming polypeptide (antigen) in the biosample, the difference in the diagnostic signal between a patient and a normal subject is maximized using the difference in the clearing system suppressing the formation of the aggregate form of the polypeptide and/or the difference in hydrophobic interaction.
(c) The present invention can be carried out in a convenient and prompt manner, and can automate a method for detecting an aggregate form of an aggregate-forming polypeptide in a biosample.

### Brief Description of the Drawings

FIG. 1A shows a change of Aβ oligomer according to an incubation time for 4 days after rec. Aβ1-42 spiking.
FIG. 1B shows a change of Aβ oligomer according to an incubation time for 6 days after rec. Aβ1-42 spiking.
FIG. 1C shows changes of Aβ oligomer according to incubation times for 2, 3, 4, and 5 days after rec. Aβ1-42 spiking.
FIG. 1D shows changes of Aβ oligomer according to the incubation for 5 days after rec. Aβ1-42 spiking and the incubation for 0 days and 5 days without rec. Aβ1-42 addition.
FIG. 2A shows a change of Aβ oligomer according to the incubation for 6 days after rec. Aβ 9-42 spiking.
FIG. 2B shows changes of Aβ oligomer according to the incubation for 2, 3, and 4 days after spiking of rec. Aβ 9-42 binding to Aβ.
FIG. 3 shows changes of α-synuclein oligomer with increased time for incubation of 0, 2, and 4 days after spiking of recombinant α-synuclein.

### Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

### EXAMPLE

### Example 1: Materials

Carbonate-Bicarbonate Buffer, PBST, TBST, and PBS were purchased from Sigma. Block Ace was purchased from Bio-rad. Buffer A was prepared by diluting Block Ace to 0.4% in TBST. A blocking buffer was prepared by diluting 1% Block Ace to 0.4% in TBST. 6E10 antibody was purchased from Biolegend. 3B6 antibody was purchased from Novus Biologicals. For 3B6-biotin antibody, the antibody purchased from Novus Biologicals was biotinylated by Peoplebio. Streptavidin-HRP was purchased from Thermo Scientific. HBR1 was purchased from Scantibodies Laboratory. FF51-HRP was purchased from The H lab. Recombinant Aβ1-42 was purchased from Biolegend. Recombinant Aβ 9-42 biotin was purchased from Anaspec. Recombinant Aβ 9-42 was purchased from Anaspec. Recombinant α-synuclein was purchased from Millipore. Plasma samples were obtained from Seoul National University Bundang Hospital and Chungang University Hospital. ECL solution was purchased from Rockland. Plates were purchased from Nunc. The epitopes to 6E10 and FF51 antibodies have the amino acid sequences including amino acids 3-8 and 1-4, respectively, of the the human Aβ peptide sequence. The epitopes to 3B6 and 3B6 biotin antibodies have sequences including 119-140 amino acid residues of the α-synuclein protein sequence.

### Example 2: Preparation of 6E10 plates

After 30 µg of 6E10 antibody (anti-Aβ protein, Biolegend) was diluted in 10 mℓ of a coating buffer (Sigma), 100 µl was dispensed into each well in a plate (Nunc), followed by reaction in a 4°C refrigerator for one day. The plate was washed three times with PBS, and 240 µl of the blocking buffer in which 1% block Ace was dissolved in D.W. was dispensed, followed by reaction at room temperature for 2 hours or more. The plate was washed three times with BPS and was then dried at room temperature for 30 minutes before.

### Example 3: Preparation of 3B6 plates

After 20 µg of 3B6 antibody (anti-α-synuclein protein, Novus Biologicals) was diluted in 10 mℓ of a coating buffer (Sigma), 100 µl was dispensed into each well in a plate (Nunc), followed by a reaction in a 4°C refrigerator for one day. The plate was washed three times with PBS, and 240 µl of the blocking buffer in which 1% block Ace was dissolved in D.W. was dispensed, followed by reaction at room temperature for 2 hours or more. The plate was washed with three times with BPS, and was then dried at room temperature for 30 minutes before.

### Example 4: Preparation of control

For a positive control, 990 µℓ of PBST was added to 10 µℓ of recombinant Aβ1-42 (rec. Aβ) (1 µg/mℓ), and 100 µℓ was used. For a positive control, 990 µℓ of PBST was added to 10 µℓ of α-synuclein (1 mg/ml), and 100 µℓ was used. For a negative control, 100 µℓ of PBS was used.

### Example 5: Preparation of samples

Samples were prepared based on two samples. Frozen plasma samples were dissolved in a 37°C heat block for 15 minutes, followed by vortexing for 30 seconds before use. For the rec. Aβ1-42 (1 ng)-spiked samples, 8.08 µℓ of HBR1 (0.123 mg/ml), 180 µℓ of PBST, and 20 µℓ of rec. Aβ1-42 (1 ng/10 ul) were mixed with 20 µℓ of plasma to prepare a total of 228.08 µℓ. For the rec. Aβ9-42 biotin (1 ng)-spiked samples, 8.08 µℓ of HBR1 (0.123 mg/ml), 180 µℓ of PBST, and 20 µℓ of rec. Aβ 9-42 biotin (1 ng/10 ul) were mixed with 20 µℓ of plasma to prepare a total of 228.08 µℓ. For the rec. Aβ9-42 (1 ng)-spiked samples, 8.08 µℓ of HBR1 (0.123 mg/ml), 180 µℓ of PBST, and 20 µℓ of rec. Aβ 9-42 (1 ng/10 ul) were mixed with 20 µℓ of plasma to prepare a total of 228.08 µℓ. For recombinant α-synuclein (1 µg)-spiked samples, 8.08 µℓ of HBR1 (0.123 mg/ml), 180 µℓ of PBST, and 20 µℓ of recombinant α-synuclein (1 pg/10 ul) were mixed with 20 µℓ of plasma to prepare a total of 228.08 µℓ. In addition, for recombinant peptide-unspiked samples, 8.08 µℓ of HBR1 (0.123 mg/ml) and 200 µℓ of PBST were mixed with 20 µℓ of plasma to prepare a total of 228.08 µℓ.

### Example 6: Incubation

In example 5, the samples prepared by treatment with rec. Aβ1-42 were incubated in a 37°C incubator for 4 days and 6 days, respectively. In example 5, the samples prepared by treatment with rec. Aβ1-42 were incubated in a 37°C incubator for 2 days, 3 days, 4 days, and 5 days, respectively. The samples prepared by treatment without rec. Aβ1-42 were incubated in a 37°C incubator for 0 days and 5 days, respectively. In addition, in example 5, the samples prepared by treatment with rec. Aβ 9-42 biotin were incubated for 6 days. In example 5, the samples prepared by treatment with rec. Aβ 9-42 were incubated for 2 days, 3 days, and 4 days, respectively. In addition, in example 5, the samples prepared by treatment with recombinant α-synuclein were incubated for 0 day, 2 days, 4 days, and 6 days, respectively.

### Example 7: Detection of Aβ oligomers in samples treated with rec. Aβ1-42 and incubated for 4 days and 6 days using multimer detection system (MDS)

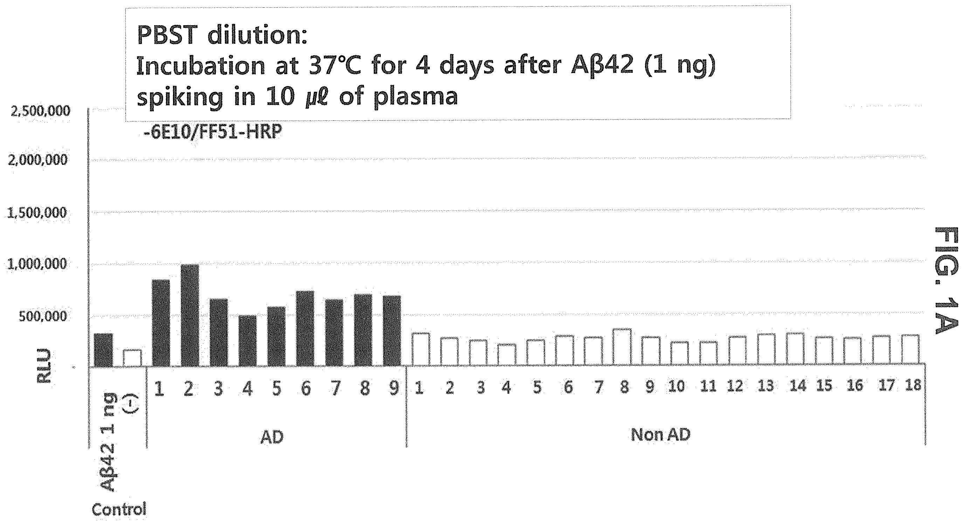

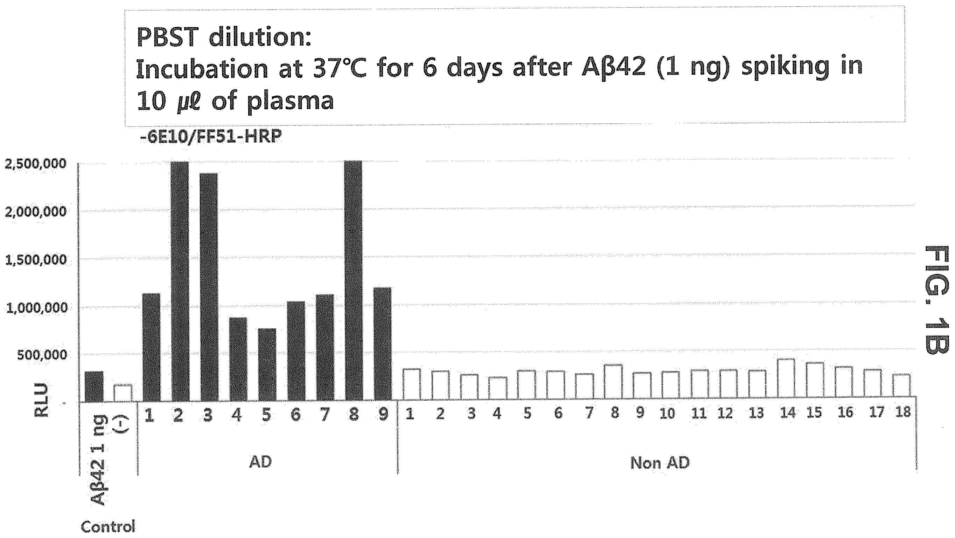

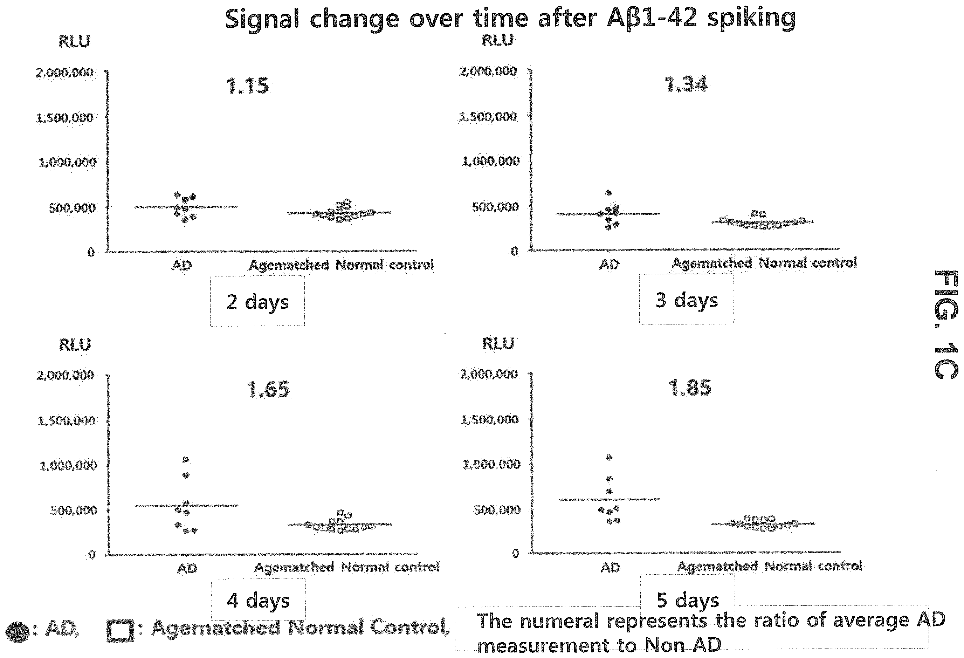

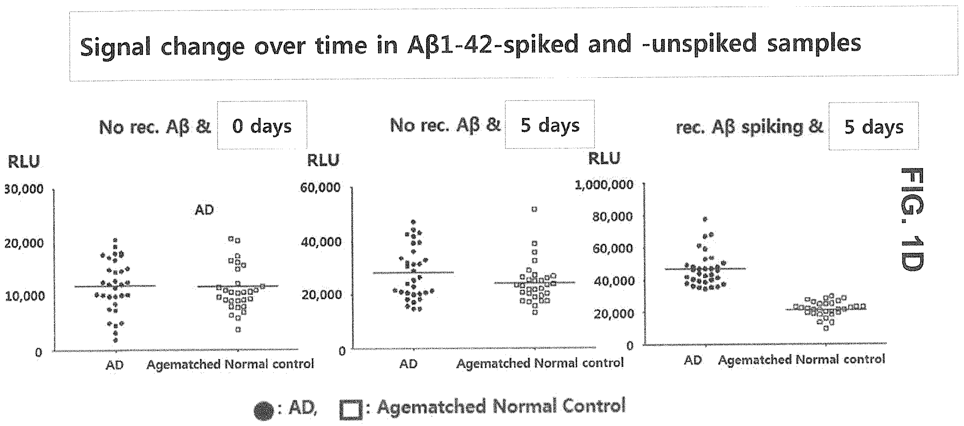

The positive control, the negative control, and the samples treated with rec. Aβ1-42 and incubated for 4 days and 6 days were dispensed in 100 µl each on 6E10 coated plate (3 µg/mℓ), followed by reaction at room temperature for 1 hour. After the plate was washed three times with TBST, the FF51-HRP antibody was diluted 1/1000 in buffer A, and then 100 ul of each was dispensed, followed by reaction at room temperature for 1 hour. The plate was washed three times with TBST, and 100 µl of the ECL solution was dispensed. The plate reacted with ELC was inserted into a luminometer (PerkinElmer) to measure a luminescent signal. The results are shown in FIG. 1.

FIGS. 1A and 1B show that the signal of the AD sample is increased compared with the signal of the Non AD sample according to the incubation time after the addition of rec. Aβ1-42. The difference between AD and Non AD is shown in each condition after incubation for 4 days and 6 days.

Considering FIGS. 1A and 1B, the reason why the signal of the Aβ oligomer was higher in the AD patient samples compared with the Non AD patient samples is considered to be that the clearing system suppressing the formation of Aβ oligomer in the AD patient samples was activated less than that in the Non AD patient samples.

### Example 8: Detection of Aβ oligomers in samples treated with rec. Aβ1-42 and incubated for 2 days, 3 days, 4 days, and 5 days using multimer detection system (MDS)

The positive control, the negative control, and the samples treated with rec. Aβ1-42 and incubated for 2 days, 3 days, 4 days, and 6 days were dispensed in 100 µl each on 6E10 coated plate (3 pg/ml), followed by reaction in a 27°C incubator in a standing state. After the plate was washed three times with TBST, the FF51-HRP antibody was added to buffer A to reach a concentration of 10 ng/ml and then 100 µℓ of each was dispensed. The plate was subjected to reaction in a 27°C incubator in a standing state for 1 hour, and washed three times with TBST, and then 100 ul of the ECL solution was dispensed. The plate reacted with ELC was inserted into a luminometer (PerkinElmer) to measure a luminescent signal. The results are shown in FIG. 1C.

FIG. 1C shows that the signal of the AD samples is increased by 1.15 times, 1.34 times, 1.65 times, and 1.84 times compared with the signal of the Non AD samples according to the incubation time for 2 days, 3 days, 4 days, and 5 days after the addition of rec. Aβ1-42. It shows that, as the number of days of incubation increased, the difference between AD and Non AD was gradually increased.

Referring to FIGS. 1C, the reason why the signal of the Aβ oligomer was higher in AD patient samples compared with Non AD patient samples is considered to be that the clearing system suppressing the formation of the Aβ oligomer in the AD patient samples was activated less than that in the Non AD patient samples.

### Example 9: Detection of Aβ oligomers in samples treated with rec. Aβ1-42 and incubated for 5 days and samples treated without rec. Aβ1-42 and incubation for 0 days and 5 days using multimer detection system (MDS)

The positive control, the negative control, and the samples treated with rec. Aβ1-42 and incubated for 5 days and the samples treated without rec. Aβ1-42 and incubated for 0 days and 5 days were dispensed in 100 µl each on 6E10 coated plate (3 µg/mℓ), followed by reaction in a 27°C incubator in a standing state. After the plate was washed three times with TBST, the FF51-HRP antibody was added to buffer A to reach a concentration of 10 ng/ml, and then 100 µℓ of each was dispensed. The plate was subjected to reaction in a 27°C incubator in a standing state for 1 hour and washed three times with TBST, and then 100 ul of the ECL solution was dispensed. The plate reacted with ELC was inserted into a luminometer (PerkinElmer) to measure a luminescent signal. The results are shown in FIG. 1D.

FIG. 1D shows Aβ oligomer measurement data in the samples prepared by the addition of rec. Aβ1-42 and incubation for 5 days and the samples prepared by the non-addition of rec. Aβ1-42 and incubation for 0 day and 5 days, and illustrates the increase states of the signal of the AD sample and the signal of the Non AD sample over time when Aβ1-42 was spiked and was not spiked, indicating that a differentiation between AD and Non AD was shown only in the samples prepared by the addition of rec. Aβ1-42 and incubation for 5 days.

Referring to FIGS. 1D, the reason why the signal of the Aβ oligomer was high in AD patient samples compared with Non AD patient samples is considered that the clearing system, which suppresses the formation of Aβ oligomer in the AD patient samples, is activated less than that in the Non AD patient samples, showing an effect of the clearing system that suppresses the formation of Aβ oligomer when Aβ1-42 was spiked.

### Example 10: Detection of Aβ oligomers in samples treated with rec. Aβ9-42 biotin and incubated for 6 days using multimer detection system (MDS)

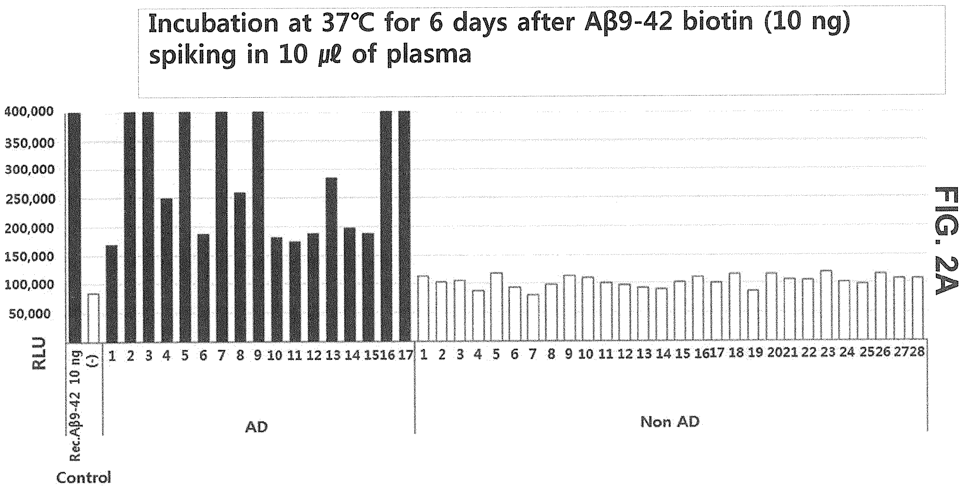

The positive control, the negative control, and the samples treated with rec. Aβ9-42 biotin and incubated for 6 days were dispensed in 100 µl each on 6E10 coated plate (3 µg/mℓ), followed by reaction at room temperature for 1 hour. After the plate was washed three times with TBST, the FF51-HRP antibody was diluted 1/1000 in buffer A, and then 100 ul of each was dispensed, followed by reaction at room temperature for 1 hour. The plate was washed three times with TBST, and 100 µl of the ECL solution was dispensed. The plate reacted with ELC was inserted into a luminometer (PerkinElmer) to measure a luminescent signal, and the results are summarized in FIG. 2a.

FIG. 2a shows that the signal of the AD patient group was higher than that of the normal group after the spiking of the rec. Aβ9-42 biotin binding to Aβ and then incubation for 6 days.

Referring to FIG. 2a, it is considered that the rec. Aβ9-42 biotin bound to Aβ to promote aggregation, resulting in changes of quantitative and size portions of antigens, leading to differentiations.

### Example 11: Detection of Aβ oligomers in samples treated with rec. Aβ9-42 biotin and incubated for 2 days, 3 days, and 4 days using multimer detection system (MDS)

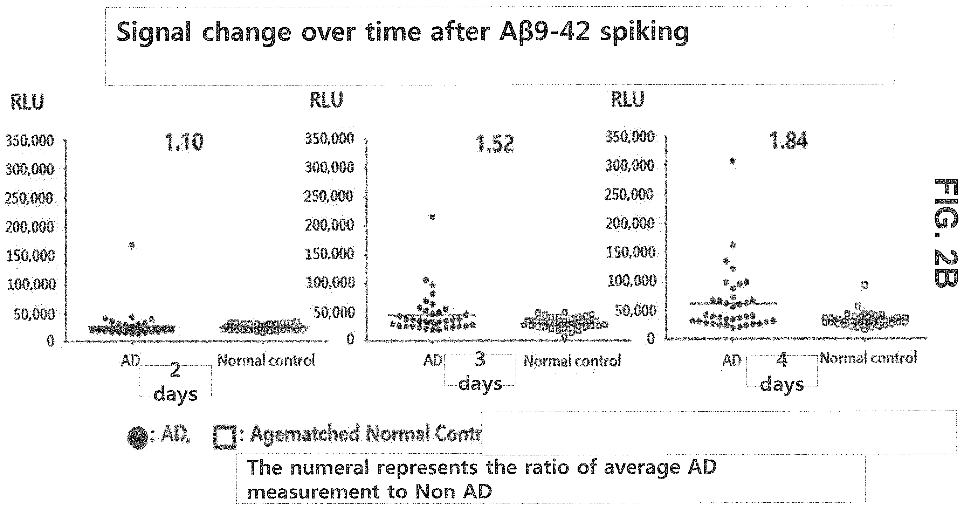

The positive control, the negative control, and the samples treated with rec. Aβ9-42 and incubated for 2 days, 3 days, and 4 days, were dispensed in 100 µl each on 6E10 coated plate (3 µg/mℓ), followed by reaction in a 27°C incubator in a standing state. After the plate was washed three times with TBST, the FF51-HRP antibody was added to buffer A to reach a concentration of 10 ng/ml, and then 100 µℓ of each was dispensed. The plate was subjected to reaction in a 27°C incubator in a standing state for 1 hour and washed three times with TBST, and then 100 ul of the ECL solution was dispensed. The plate reacted with ELC was inserted into a luminometer (PerkinElmer) to measure a luminescent signal, and the results are summarized in FIG. 2b.

FIG. 2b shows that, when rec. Aβ9-42 binding to Aβ was spiked and incubated for 2 days, 3 days, and 4 days, the signal of the AD patient group was 1.1 times, 1.52 times, and 1.84 times higher than the signal of the normal group with the increased time.

Referring to FIG. 2b, it is considered that rec. Aβ9-42 biotin bound to Aβ to promote aggregation, resulting in changes of quantitative and size portions of antigens, leading to differentiations.

### Example 12: Detection of α-synuclein oligomers in samples treated with recombinant α-synuclein and incubated for 0 days, 2 days, 4 days, and 6 days using multimer detection system (MDS)

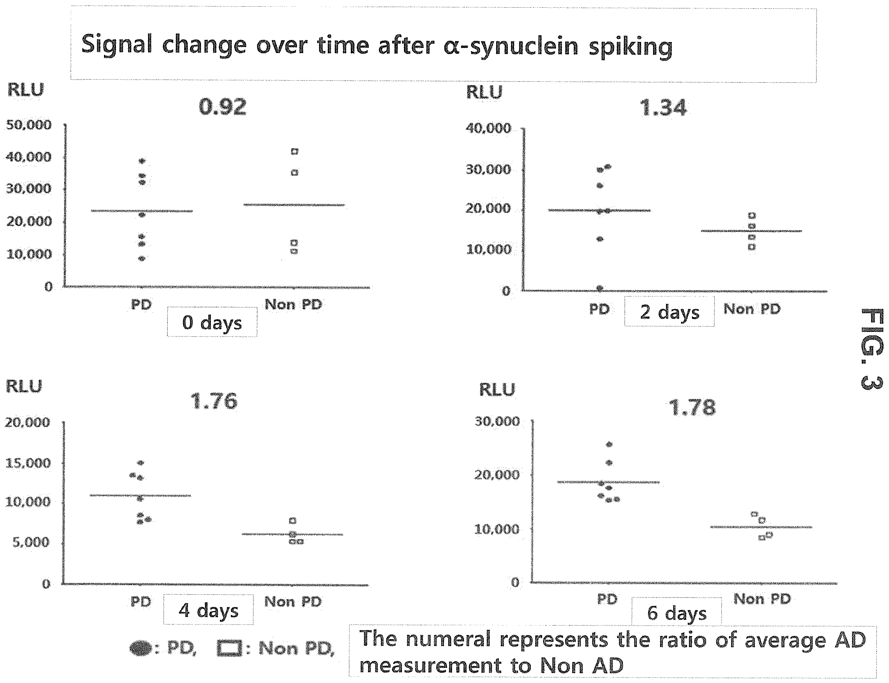

The positive control, the negative control, and the samples treated with α-synuclein and incubated for 0 days, 2 days, 4 days, and 6 days, were dispensed in 100 µl each on 3B6 coated plate (2 µg/mℓ), followed by reaction in a 27°C incubator in a standing state. After the plate was washed three times with TBST, the 3B6-biotin antibody was added to buffer A to reach 2 ug/ml, and then 100 µℓ of each was dispensed. The plate was reacted in a 27°C incubator in a standing state for 1 hour, and washed three times with TBST. Streptavidin-HRP was diluted 1/5000 in buffer A, and 100 µℓ each was dispensed. Thereafter, the plate was reacted in a 27°C incubator in a standing state for 1 hour and washed three times with TBST. 100 µl of the ECL solution was dispensed. The plate reacted with ELC was inserted into a luminometer (PerkinElmer) to measure a luminescent signal, and the results are summarized in FIG. 3.

FIG. 3 shows signal change data of the signal of the PD sample and the signal of the Non PD sample with increased time after the addition of α-synuclein and incubation for 0 days, 2 days, 4 days, and 6 days, and illustrates that the ratio of the signal of the PD sample to the signal of the Non PD sample was 9.2 times on day 0, increased to 1.34 times, 1.76 times, and 1.78 times for the incubation for 2 days, 4 days, and 6 days.

Referring to FIG. 3, the reason why the signal of α-synuclein was higher in the PD patient samples compared with the Non AD patient samples is considered to be that the clearing system suppressing the formation of α-synuclein oligomer in the PD patient samples was activated less than that in the Non PD patient samples.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims.

## Claims

1. A method for detecting an aggregate form of an aggregate-forming polypeptide in a biosample, the method comprising the steps of:
(a) spiking, with a biosample to be analyzed, (i) a monomeric or multimeric form of the aggregate-forming polypeptide, (ii) a hydrophobic deleted derivative of the aggregate-forming polypeptide, or (iii) a monomeric or multimeric form of the aggregate-forming polypeptide and a hydrophobic deleted derivative of the aggregate-forming polypeptide;
(b) additionally forming an aggregate form of the aggregate-forming polypeptide by incubating a product of step (a) at 25-40°C for 2-6 days;
(c) contacting, with a product of step (b), a binder-label in which a signal generation label is conjugated to a binder binding to the aggregate form of the aggregate-forming polypeptide; and
(d) detecting a signal generated from the binder-label bound to the aggregate form of the aggregate-forming polypeptide,
wherein the incubating in step (b) is carried out for a sufficient incubation time for multimerization of the spiked (i), (ii), or (iii) by the biosample,
wherein the biosample is plasma, and
wherein the biosample for performing the multimerization of the spiked (i), (ii), or (iii) is a biosample of a human being having a disease involving the multimeric form of the aggregate-forming polypeptide.

2. The method of claim 1, wherein the aggregate-forming polypeptide is selected from the group consisting of Aβ peptide, tau protein, prion, α-synuclein, Ig light chain, serum amyloid A, transthyretin, cystatin C, β2-microglobulin, huntingtin, superoxide dismutase, serpin, and amylin.

3. The method of claim 2, wherein the aggregate-forming polypeptide is Aβ peptide, tau protein, or α-synuclein.

4. The method of claim 1, wherein the monomeric form of the aggregate-forming polypeptide is Aβ peptide including the amino acid sequence of SEQ ID NO: 1 or α-synuclein including the amino acid sequence of SEQ ID NO: 2.

5. The method of claim 1, wherein the hydrophobic deleted derivative of the aggregate-forming polypeptide is Aβ_{delete} peptide including the 37th amino acid residue to the 42nd amino acid residue in the amino acid sequence of SEQ ID NO: 1.

6. The method of claim 5, wherein the Aβ_{delete} peptide is a peptide including the 29th amino acid residue to the 42nd amino acid residue in the amino acid sequence of SEQ ID NO: 1.

7. The method of claim 6, wherein the Aβ_{delete} peptide is a peptide including the 9th amino acid residue to the 42nd amino acid residue in the amino acid sequence of SEQ ID NO: 1.

8. The method of claim 1, wherein steps (c) and (d) are performed by comprising the following steps:
(c-1) contacting the product of step (b) with a capture antibody recognizing an epitope on the aggregate-forming polypeptide capturing the aggregate form;
(c-2) contacting the captured aggregate form with a detection antibody recognizing an epitope on the aggregate-forming polypeptide; and
(c-3) detecting an aggregate form-detection antibody complex.

9. The method of claim 8, wherein the detection antibody is a detection antibody recognizing an epitope identical to or overlapped with the epitope in step (c-1).

10. The method of claim 8, wherein the capture antibody is bound to a solid substrate.

11. The method of claim 8, wherein the detection antibody has a label generating a detectable signal.

12. The method of claim 11, wherein the label bound to the detection antibody includes a compound label, an enzyme label, a radioactive label, a fluorescent label, a luminescent label, a chemiluminescent label, and an FRET label.

## Patentansprüche

1. Verfahren zum Nachweis einer Aggregatform eines aggregatbildenden Polypeptids in einer Bioprobe, wobei das Verfahren die Schritte umfasst:
(a) Dotieren ("Spiking") mit einer zu analysierenden Bioprobe (i) einer monomeren oder multimeren Form des aggregatbildenden Polypeptids, (ii) eines hydrophob deletierten Derivats des aggregatbildenden Polypeptids oder (iii) einer monomeren oder multimeren Form des aggregatbildenden Polypeptids und eines hydrophob deletierten Derivats des aggregatbildenden Polypeptids;
(b) zusätzlich Bilden einer Aggregatform des aggregatbildenden Polypeptids durch Inkubieren eines Produkts aus Schritt (a) bei 25-40°C für 2-6 Tage;
(c) Inkontaktbringen mit einem Produkt aus Schritt (b) einer Bindermarkierung, bei der eine Signalerzeugungsmarkierung an einen Binder konjugiert ist, der an die Aggregatform des aggregatbildenden Polypeptids bindet; und
(d) Nachweisen eines Signals, das von der Binder-Markierung erzeugt wird, die an die Aggregatform des aggregatbildenden Polypeptids gebunden ist,
wobei das Inkubieren in Schritt (b) für eine ausreichende Inkubationszeit zur Multimerisierung der dotierten (i), (ii) oder (iii) durch die Bioprobe durchgeführt wird,
wobei die Bioprobe Plasma ist, und
wobei die Bioprobe zur Durchführung der Multimerisierung der dotierten (i), (ii) oder (iii) eine Bioprobe eines Menschen ist, der an einer Krankheit leidet, an der die multimere Form des aggregatbildenden Polypeptids beteiligt ist.

2. Verfahren nach Anspruch 1, wobei das aggregatbildende Polypeptid ausgewählt ist aus der Gruppe bestehend aus Aß-Peptid, Tau-Protein, Prion, α-Synuclein, Ig-Leichtkette, Serum-Amyloid A, Transthyretin, Cystatin C, β2-Mikroglobulin, Huntingtin, Superoxiddismutase, Serpin und Amylin.

3. Verfahren nach Anspruch 2, wobei das aggregatbildende Polypeptid Aβ-Peptid, Tau-Protein oder α-Synuclein ist

4. Verfahren nach Anspruch 1, wobei die monomere Form des aggregatbildenden Polypeptids ein Aß-Peptid ist, das die Aminosäuresequenz von SEQ ID NO: 1 enthält, oder α-Synuclein, das die Aminosäuresequenz von SEQ ID NO: 2 enthält.

5. Verfahren nach Anspruch 1, wobei das hydrophobe deletierte Derivat des aggregatbildenden Polypeptids Aβ_{delete}-Peptid ist, das den 37. Aminosäurerest bis zum 42. Aminosäurerest in der Aminosäuresequenz von SEQ ID NO: 1 umfasst.

6. Verfahren nach Anspruch 5, wobei das Aβ_{delete}-Peptid ein Peptid ist, das den 29. Aminosäurerest bis zum 42. Aminosäurerest in der Aminosäuresequenz von SEQ ID NO: 1 umfasst.

7. Verfahren nach Anspruch 6, wobei das Aβ_{delete}-Peptid ein Peptid ist, das den 9. Aminosäurerest bis zum 42. Aminosäurerest in der Aminosäuresequenz von SEQ ID NO: 1 umfasst.

8. Verfahren nach Anspruch 1, wobei die Schritte (c) und (d) durchgeführt werden, indem sie die folgenden Schritte umfassen:
(c-1) Kontaktieren des Produkts aus Schritt (b) mit einem Fangantikörper, der ein Epitop auf dem aggregatbildenden Polypeptid erkennt und die Aggregatform einfängt;
(c-2) Kontaktieren der eingefangenen Aggregatform mit einem Detektionsantikörper, der ein Epitop auf dem aggregatbildenden Polypeptid erkennt; und
(c-3) Nachweis eines Aggregatform-Nachweis-Antikörper-Komplexes.

9. Verfahren nach Anspruch 8, wobei der Detektionsantikörper ein Detektionsantikörper ist, der ein Epitop erkennt, das mit dem Epitop in Schritt (c-1) identisch ist oder sich damit überlappt.

10. Verfahren nach Anspruch 8, wobei der Fangantikörper an ein festes Substrat gebunden ist.

11. Verfahren nach Anspruch 8, wobei der Detektionsantikörper eine Markierung aufweist, die ein nachweisbares Signal erzeugt.

12. Verfahren nach Anspruch 11, wobei die an den Detektionsantikörper gebundene Markierung eine Verbindungsmarkierung, eine Enzymmarkierung, eine radioaktive Markierung, eine fluoreszierende Markierung, eine lumineszierende Markierung, eine chemilumineszierende Markierung und eine FRET-Markierung umfasst.

## Revendications

1. Procédé pour détecter une forme agrégée d'un polypeptide formant un agrégat dans un échantillon biologique, le procédé comprenant les étapes suivantes :
(a) l'ensemencement, avec un échantillon biologique devant être analysé, (i) d'une forme monomère ou multimère du polypeptide formant un agrégat, (ii) d'un dérivé à délétion hydrophobe du polypeptide formant un agrégat, ou (iii) d'une forme monomère ou multimère du polypeptide formant un agrégat et d'un dérivé à délétion hydrophobe du polypeptide formant un agrégat ;
(b) de plus la formation d'une forme agrégée du polypeptide formant un agrégat par incubation du produit de l'étape a) à 25 à 40°C pendant 2 à 6 jours ;
(c) la mise en contact, avec un produit de l'étape (b), d'un lieur-marqueur dans lequel un marqueur générant un signal est couplé à un lieur se liant à la forme agrégée du polypeptide formant un agrégat ; et
(d) la détection d'un signal généré par le lieur-marqueur lié à la forme agrégée du polypeptide formant un agrégat,
dans lequel l'incubation dans l'étape (b) effectué pendant un temps d'incubation suffisant pour une multimérisation du (i), (ii) ou (iii) ensemencé par l'échantillon,
dans lequel l'échantillon biologique est un plasma, et
dans lequel l'échantillon biologique pour effectuer la multimérisation du (i), (ii) ou (iii) ensemencé est un échantillon biologique d'un être humain ayant une maladie impliquant la forme multimère du polypeptide formant un agrégat.

2. Procédé selon la revendication 1, dans lequel le polypeptide formant un agrégat est choisi dans le groupe constitué par le peptide Ab, la protéine tau, un prion, l'a-synucléine, une chaîne légère d'Ig, l'amyloïde sérique A, la transthyrétine, la cystatine C, la microglobuline b2, la huntingtine, la superoxyde dismutase, la serpine, et l'amyline.

3. Procédé selon la revendication 2, dans lequel le polypeptide formant un agrégat est le peptide Ab, la protéine tau, ou l'a-synucléine.

4. Procédé selon la revendication 1, dans lequel la forme monomère du polypeptide formant un agrégat est le peptide Ab comprenant la séquence d'acides aminés de la SEQ ID NO : 1 ou l'a-synucléine comprenant la séquence d'acides aminés de la SEQ ID NO : 2.

5. Procédé selon la revendication 1, dans lequel le dérivé à délétion hydrophobe du polypeptide formant un agrégat est le peptide Ab_{delete} comprenant du 37ème résidu d'acide aminé au 42ème résidu d'acide aminé dans la séquence d'acides aminés de la SEQ ID NO : 1.

6. Procédé selon la revendication 5, dans lequel le peptide Ab_{delete} est un peptide comprenant du 29ème résidu d'acide aminé au 42ème résidu d'acide aminé dans la séquence d'acides aminés de la SEQ ID NO : 1.

7. Procédé selon la revendication 6, dans lequel le peptide Ab_{delete} est un peptide comprenant du 9ème résidu d'acide aminé au 42ème résidu d'acide aminé dans la séquence d'acides aminés de la SEQ ID NO : 1.

8. Procédé selon la revendication 1, dans lequel les étapes (c) et (d) sont effectuées en comprenant les étapes suivantes :
(c-1) mise en contact du produit de l'étape (b) avec un anticorps de capture reconnaissant un épitope sur le polypeptide formant un agrégat capturant la forme agrégée ;
(c-2) mise en contact de la forme agrégée capturée avec un anticorps de détection comprenant un épitope sur le polypeptide formant un agrégat ; et
(c-3) détection d'un complexe de forme agrégée-anticorps de détection.

9. Procédé selon la revendication 8, dans lequel l'anticorps de détection est un anticorps de détection reconnaissant un épitope identique à ou en chevauchement avec l'épitope dans l'étape (c-1).

10. Procédé selon la revendication 8, dans lequel l'anticorps de capture est lié à un substrat solide.

11. Procédé selon la revendication 8, dans lequel l'anticorps de détection a un marqueur générant un signal détectable.

12. Procédé selon la revendication 11, dans lequel le marqueur lié à l'anticorps de détection comprend un marqueur de type composé, un marqueur enzymatique, un marqueur radioactif, un marqueur fluorescent, un marqueur luminescent, un marqueur chimioluminescent, et un marqueur FRET.
